# EUROPEAN PATENT APPLICATION

(11) **EP 4 428 230 A1**
(43) Date of publication of application: **11.09.2024**
(21) Application number: 22889965.4
(22) Date of filing: 02.11.2022
(51) Int. Cl.: C12N 5/079, C12Q 1/02, C12N 15/12

(54) **CULTURE METHOD, CULTURE PRODUCT, SPHEROID, AND METHOD FOR SCREENING FOR TEST SUBSTANCE**

(30) Priority: 05.11.2021 JP 2021180737
(71) Applicant: JSR Corporation, Tokyo 105-8640 (JP); Keio University, Tokyo, 108-8345 (JP)
(72) Inventor: HIRAMINE Hayato, Tokyo 105-8640 (JP); NAGASHIMA Ryosuke, Tokyo 105-8640 (JP); MAEDA Sumihiro, Tokyo 160-8582 (JP); OKANO Hideyuki, Tokyo 160-8582 (JP); ISHIKAWA Mitsuru, Tokyo 160-8582 (JP)
(74) Representative: TBK
(86) International application number: PCT/JP2022/040950
(87) International publication number: WO 2023/080145

(57) **Abstract**

A culture method includes culturing a spheroid of a human neural cell-like cell in the presence of a tau protein aggregate and culturing a culture product in a culture medium containing 5 µg/mL or greater of a lipid, in which the lipid is one or more selected from the group consisting of a glycerolipid, a glycerophospholipid, and a sphingolipid. The culture method includes culturing a spheroid of a human neural cell-like cell in the presence of a tau protein aggregate and culturing a culture product in a culture medium containing a neurotrophic factor.

## Description

### [Technical Field]

The present invention relates to a method for culturing human neural cell-like cells, a culture product, a spheroid, and a method for screening a test substance.

Priority is claimed on Japanese Patent Application No. 2021-180737, filed November 5, 2021, the content of which is incorporated herein by reference.

### [Background Art]

A tau protein is a microtubule-associated protein expressed in nerve cells and glial cells of the central nervous system and the peripheral nervous system, and the tau protein is known to contribute to the stabilization of microtubules. Further, the tau protein is also known to be involved in physiological phenomena of the nervous system such as nerve signal transduction in addition to the stabilization of microtubules.

Neurofibrillary tangles (NFTs), which are frequently found in the brains of patients with dementia, are known to occur such that tau proteins are dissociated from microtubules due to excessive phosphorylation or the like of the tau proteins, and misfolded tau proteins are generated in nerve cells and accumulated in nerve cells. Therefore, nerve cells to be used in vitro tests to elucidate neurodegeneration mechanisms or to develop novel dementia drugs are required to be nerve cells in which misfolded tau proteins are accumulated.

As a method for obtaining nerve cells in which misfolded tau proteins are accumulated, a method of culturing nerve cells by using an exogenous tau protein aggregate (tau-seed) is known (for example, see Non-Patent Document 1 and Non-Patent Document 2).

### [Citation List]

### [Non-Patent Document]

[Non-Patent Document 1]
   Karikari TK et al., "Distinct Conformations, Aggregation and Cellular Internalization of Different Tau Strains.", Front Cell Neurosci., Vol. 13, Article 296, 2019, https://doi.org/10.3389/fncel.2019.00296.
[Non-Patent Document 2]
   Reillya P et al., "Novel human neuronal tau model exhibiting neurofibrillary tangles and transcellular propagation", Neurobiol Dis., Vol. 106, pp. 222-234, 2017.

### [Summary of Invention]

### [Technical Problem]

However, nerve cells in which misfolded tau proteins are accumulated are difficult to prepare efficiently with the culture methods described in Non-Patent Documents 1 and 2.

The present invention has been made in consideration of the above-described circumstances, and an object thereof is to provide a culture method that enables efficient preparation of human neural cell-like cells in which misfolded tau proteins are accumulated by tau-seeding.

### [Solution to Problem]

That is, the present invention includes the following aspects.
(1) A culture method including: culturing a spheroid of a human neural cell-like cell in the presence of a tau protein aggregate and culturing a culture product in a culture medium containing 5 µg/mL or greater of a lipid, in which the lipid is one or more selected from the group consisting of a glycerolipid, a glycerophospholipid, and a sphingolipid.
(2) The culture method according to (1), in which the lipid is formed of a glycerolipid, a glycerophospholipid, and a sphingolipid.
(3) The culture method according to (1) or (2), in which a concentration of a sterol lipid contained in the culture medium is 5 µg/mL or less.
(4) The culture method according to any one of (1) to (3), in which the lipid is one or more selected from the group consisting of triacylglycerol, lysophosphatidylcholine, phosphatidylcholine, phosphatidic acid, and sphingomyelin.
(5) The culture method according to any one of (1) to (4), in which a concentration of the lipid contained in the culture medium is 5 µg/mL or greater and 150 µg/mL or less.
(6) The culture method according to any one of (1) to (5), in which the culture medium further contains a neurotrophic factor.
(7) A culture method including: culturing a spheroid of a human neural cell-like cell in a presence of a tau protein aggregate and culturing a culture product in a culture medium containing a neurotrophic factor.
(8) The culture method according to (6) or (7), in which the neurotrophic factor is a brain-derived neurotrophic factor or a glial cell line-derived neurotrophic factor.
(9) The culture method according to any one of (1) to (8), in which the human neural cell-like cell has a mutation in a microtubule-associated protein tau gene.
(10) The culture method according to (9), in which in the mutation, a mutation of a base sequence in an exon is a mutation of a base sequence that encodes one or more amino acid mutations selected from the group consisting of N279K, V337M, P301L, P301S, and R406W, and a mutation of a base sequence in intron 10 is a mutation of one or more base sequences selected from the group consisting of E10+14 and E10+16.
(11) The culture method according to any one of (1) to (10), in which the tau protein aggregate is an aggregate of a recombinant tau protein.
(12) The culture method according to (11), in which the recombinant tau protein is expressed from a microtubule-associated protein tau gene having a mutation of a base sequence of exon 10.
(13) The culture method according to any one of (1) to (12), in which the human neural cell-like cell is a culture product.
(14) A culture product cultured by the culture method according to any one of (1) to (6).
(15) A culture product cultured by the culture method according to any one of (7) to (13).
(16) A spheroid containing a human neural cell-like cell, including: a MC1-positive tau protein in a cell.
(17) A method for screening a test substance, including: bringing the culture product according to (14) or (15) or the spheroid containing a human neural cell-like cell according to (16) into contact with the test substance and evaluating an influence of the human neural cell-like cell.

### [Advantageous Effects of Invention]

According to the culture method of the above-described aspect, it is possible to efficiently prepare human neural cell-like cells in which misfolded tau proteins are accumulated.

### [Brief Description of Drawings]

FIG. 1A is a photograph showing the results of Western blotting in Experimental Example 6.
FIG. 1B is a signal quantification graph of Western blotting in Experimental Example 6.
FIG. 1C is a signal quantification graph of Western blotting in Experimental Example 6.
FIG. 2A is a fluorescent immunostaining image of a section of a spheroid using an MC1 antibody in Experimental Example 7.
FIG. 2B is a fluorescent immunostaining image of a section of a spheroid using an RTM 38 antibody in Experimental Example 7.
FIG. 2C is a fluorescent immunostaining image of a section of a spheroid using DAPI in Experimental Example 7.
FIG. 3 is a fluorescent immunostaining image obtained by merging FIGS. 2A to 2C in Experimental Example 7.
FIG. 4 shows fluorescent immunostaining image of sections of spheroids in Experimental Example 8.
FIG. 5 shows fluorescent immunostaining image of sections of spheroids in Experimental Example 9.
FIG. 6 is an optical microphotograph showing the state of spheroids in a 96-well culture plate after the addition of tau-seeds and after being subjected to suspension culture for 8 weeks in Experimental Example 10.
FIG. 7 is a graph showing an average value of sizes of 24 spheroids and a coefficient of variation (CV) of the sizes of 24 spheroids in Experimental Example 10.
FIG. 8A is an immunoelectron microscopic image of spheroids recovered in Experimental Example 5, in Experimental Example 11.
FIG. 8B is an immunoelectron microscopic image of a brain section of a patient with Alzheimer's disease (AD) in Experimental Example 11.
FIG. 9 is a bright-field image of spheroids in Experimental Example 12.
FIG. 10 is a fluorescent immunostaining image of a spheroid cultured in a culture medium 6 using an MC1 antibody in Experimental Example 12.
FIG. 11 is a fluorescent immunostaining image of a spheroid cultured in a culture medium 7 using an MC1 antibody in Experimental Example 12.
FIG. 12 is a fluorescent immunostaining image of a spheroid cultured in a culture medium 8 using an MC1 antibody in Experimental Example 12.

### [Description of Embodiments]

Hereinafter, the present invention will be described in more detail with reference to embodiments, but the present invention is not limited to the following embodiments.

Each component described in the present specification, for example, the component contained in the culture medium and the component used in each step can be used alone or in combination of two or more kinds thereof unless otherwise specified.

In the present specification, a notation showing a numerical value range such as "1 to 1000" has the same meaning as "1 or greater and 1000 or less". In addition, a notation including a plurality of the terms "or greater" and "or less" such as "1 to 1000 and preferably 10 to 100" has the same meaning as "1 or greater and 1000 or less, 1 or greater and 100 or less, 10 or greater and 1000 or less, or 10 or greater and 100 or less".

In the present specification, the spheroid denotes a mass formed by the adhesion of two or more cells.

### «Culture method»

A culture method according to the present embodiment (hereinafter, also simply referred to as "culture method according to the present embodiment") includes culturing a spheroid of a human neural cell-like cell in the presence of tau-seeds (hereinafter, also referred to as "step A1") and culturing a culture product in a culture medium (hereinafter, also referred to as "culture medium A2") containing 5 µg/mL of greater of a lipid (hereinafter, also referred to as "step A2").

Alternatively, the culture method according to the present embodiment includes culturing a spheroid of a human neural cell-like cell in the presence of tau-seeds (hereinafter, also referred to as "step A1‴) and culturing a culture product in a culture medium (hereinafter, also referred to as "culture medium A2‴) containing a neurotrophic factor (hereinafter, also referred to as "step A2‴).

The present inventors assumed that since nerve cells are immature and the number of mature cells is small, a large number of nerve cells undergo cell death due to the toxicity of tau-seeds as the reason why the number of nerve cells in which misfolded tau proteins are accumulated is difficult to increase in the culture method using tau-seeds. Based on this assumption, it was found that nerve cells in which misfolded tau proteins are accumulated can be efficiently prepared by culturing the nerve cells by using a culture medium containing a specific amount of a lipid and a neurotrophic factor in order to mature the nerve cells and increase the number of nerve cells, thereby completing the present invention.

Examples of the human neural cell-like cell include a human nerve cell-like cell, a human neural stem cell-like cell, a human neural progenitor cell-like cell, and a human glial cell-like cell. The spheroid of the human neural cell-like cells contains at least human nerve cell-like cells and further contains preferably at least any of human neural stem cell-like cells or human neural progenitor cell-like cells. The human neural cell-like cells include a culture product. The human nerve cell-like cell denotes a cell which has a morphology unique to a nerve cell, specifically, a dendrite and an axon, and expresses a marker gene or a marker protein of a nerve cell. It is preferable that the marker of a nerve cell express, particularly, a telencephalic marker such as FOXG1 (also referred to as BF1) or SIX3. Further, the telencephalic marker is not limited to those described above. FOXG1 and SIX3 are also forebrain markers.

Next, each step of the culture method according to the present embodiment will be described in detail.

### <Step A1>

In step A1, the human neural cell-like cells are cultured in the presence of tau-seeds.

Tau-seed denotes an aggregate in which at least one or two or more kinds selected from a tau protein, a fragment of the tau protein, a recombinant tau protein, and a fragment of the recombinant tau protein are aggregated.

Among these, a tau-seed containing a recombinant tau protein or a fragment of the recombinant tau protein is preferable. In addition, the tau-seed can contain an oligomer or filament of the tau protein or recombinant tau protein. Examples of the filament thereof include a paired helical filament and a straight filament.

The MAPT gene mutation encoding a recombinant tau protein is preferably at least one or more base sequence mutation in exon 9 to exon 13.

Examples thereof include a base sequence mutation in exon 9, such as a base sequence mutation encoding an amino acid mutation of K257V, K257T, I260V, L266V, or G272V; a base sequence mutation in exon 10, such as a base sequence mutation encoding an amino acid mutation of N279K, ΔK280, L284L, N296N, N296H, ΔN296, P301L, P301S, P301T, G303V, S305N, or S305S; a base sequence mutation in exon 11, such as a base sequence mutation encoding an amino acid mutation of L315L, L315R, S320Y, S310F; a base sequence mutation in exon 12, such as a base sequence mutation encoding an amino acid mutation of V337M, E342V, G335V, G335S, Q336R, or K369I; and a base sequence mutation in exon 13, such as a base sequence mutation encoding an amino acid mutation of G389R or R406W. Among these, a base sequence mutation of exon 10 is preferable, where P301L is preferable.

The spheroid of human neural cell-like cells is cultured in the presence of tau-seeds usually in the amount of 0.0005 ng to 0.4 ng, preferably in a range of 0.001 ng to 0.2 ng, and more preferably in a range of 0.002 ng to 0.1 ng per cell contained in the spheroid of the human neural cell-like cells.

In step A1, the tau-seeds can be used together with a protein introduction reagent.

Examples of the protein introduction reagent include Effectene Transfection Reagent (catalog number "301425", QIAGEN N.V.), TransFast I Transfection Reagent (catalog number "E2431 ", Promega Corporation), TfxTM-20 Reagent (catalog number "E2391", Promega Corporation), SuperFect Transfection Reagent (catalog number "301305" QIAGEN N.V.), PolyFect Transfection Reagent (catalog number "301105", QIAGEN N.V.), LipofectAMINE (registered trade name) 2000 Reagent (catalog number "11668-019", Thermo Fisher Scientific, Inc.), LipofectAMINE (registered trade name) 3000 Reagent (catalog number "L3000015", Thermo Fisher Scientific, Inc.), JetPEI (x4) conc. (catalog number "101-30", Polyplus-transfection SA), and ExGen 500 (catalog number "R0511", Fermentas).

It is preferable that the amount of the reagent is set to be the same as the mass of tau-seeds or set to the mass within ±90% thereof.

The culture medium used for the culture in step A1 (hereinafter, also referred to as "culture medium A1 ") is usually prepared by adding a culture medium supplement, an antibacterial agent, and the like to a basal culture medium.

Examples of the basal culture medium include BME culture medium, BGJb culture medium, CMRL 1066 culture medium, Glasgow MEM (GMEM) culture medium, Improved MEM Zinc Option culture medium, 1MDM culture medium, Medium 199 culture medium, Eagle MEM culture medium, αMEM culture medium, DMEM culture medium, F-12 culture medium, DMEM/F12 culture medium, IMDM/F12 culture medium, Ham's culture medium, RPMI 1640 culture medium, Neurobasal (registered trademark) culture medium (catalogue number: "21103049", Thermo Fisher Scientific, Inc.), and Fischer's culture medium (all including trade names); and mixed culture media of these.

In the present specification, the basal culture medium refers to a culture medium that includes amino acids, antioxidants, minerals, and carbon sources such as glucose, and optionally includes serum, fatty acids, proteins such as insulin or transferrin, and the like.

The culture medium 1 can further contain a culture medium supplement and an antibacterial agent.

Examples of the culture medium supplement include L-glutamic acid, a glutamic acid-containing supplement such as "GlutaMax series" (product name, manufactured by Thermo Fisher Scientific, Inc.), an aqueous solution of amino acids such as "MEM Non-Essential Amino Acids Solution" (product name, manufactured by Thermo Fisher Scientific, Inc.), 2-mercaptoethanol, "KnockOut Serum Replacement" (product name, manufactured by Thermo Fisher Scientific, Inc.), Chemically Defined Lipid Concentrate (product name, manufactured by Thermo Fisher Scientific, Inc.), B-27 Supplement (product name, manufactured by Thermo Fisher Scientific, Inc.) including serum-derived proteins such as insulin and albumin, serum, serum substitutes, biotin, cholesterol, linoleic acid, linolenic acid, progesterone, putrescine, retinol, retinyl acetate, sodium selenite, triiodothyronine (T3), DL-α-tocopherol (vitamin E), albumin, insulin, and transferrin; and nerve supplements such as N2 Supplement including human transferrin, bovine insulin, progesterone, putrescine, and sodium selenite.

Examples of the antibacterial agent include a penicillin-based antibiotic substance, a cephem-based antibiotic substance, a macrolide-based antibiotic substance, a tetracycline-based antibiotic substance, a fosfomycin-based antibiotic substance, an aminoglycoside-based antibiotic substance, and a new quinolone-based antibiotic substance.

As the culture medium A1, a culture medium containing a carbon source such as a protein introduction reagent (10 ng/mL to 8 µg/mL), an amino acid (500 ng/mL to 2000 µg/mL), an antioxidant (100 ng/mL to 4 µg/mL), a mineral (500 ng/mL to 15 µg/mL), or glucose (500 ng/mL to 6 µg/mL) is preferable, and a culture medium containing a protein such as a fatty acid (500 ng/mL to 6 µg/mL), albumin (100 ng/mL to 2500 µg/mL), or insulin or transferrin (100 ng/mL to 300 µg/mL), an antibacterial agent (10 ng/mL to 170 µg/mL), and a reducing agent (100 ng/mL to 10 µg/mL) in addition to the above-described components is more preferable.

Suspension culture is preferable as the culture in step A1.

The suspension culture refers to carrying out culture while maintaining a state in which culture cells are suspended in a culture solution under the conditions that the culture cells are not allowed to adhere to the culture surface of a culture container (hereinafter, also referred to as "culture vessel") and a method of carrying out the culture. In the suspension culture, it is preferable to carry out three-dimensional culture.

A culture vessel of which the culture surface is cell non-adhesive is preferable as the culture vessel that is used in the suspension culture. Examples of the culture vessel of which the culture surface is cell non-adhesive include culture vessels such as a 24-well plate, a 48-well plate, a 96-well plate, and a stack plate, where the surface of the culture surface thereof has been subjected to a cell non-adhesive treatment with a 2-methacryloyloxyethyl phosphorylcholine polymer or the like or has been processed to have an uneven shape. A V-bottom or U-bottom culture vessel formed such that the cultured cells can aggregate with each other is preferable as the culture vessel that is used in the suspension culture.

The culture period of step A1 is usually 1 day or longer, preferably in a range of 3 days to 14 days, and more preferably in a range of 4 days to 12 days.

The culture of step A1 is carried out in an environment where the temperature is usually in a range of 30°C to 50°C, preferably in a range of 32°C to 48°C, and more preferably in a range of 34°C to 46°C and the carbon dioxide content proportion is usually in a range of 1% by volume to 15% by volume, preferably in a range of 2% by volume to 14% by volume, and more preferably in a range of 3% by volume to 13% by volume. In addition, it is also possible to carry out culture in an atmosphere with a high oxygen concentration.

### <Step A2>

In step A2, the culture product of step A1 is cultured in a culture medium A2 containing 5 µg/mL or greater of a lipid.

The concentration of the lipid In the culture medium A2 is 5 µg/mL or greater, 10 µg/mL or greater, 15 µg/mL or greater, 25 µg/mL or greater, 30 µg/mL or greater, 35 µg/mL or greater, 40 µg/mL or greater, or 45 µg/mL or greater. In a case where the concentration of the lipid is greater than or equal to the above-described lower limits, the maturation of nerve cell-like cells can be promoted, and the number of cells can be increased.

The upper limit of the concentration of the lipid in the culture medium is 2000 µg/mL or less, 1000 µg/mL or less, 500 µg/mL or less, 150 µg/mL or less, or 100 µg/mL or less.

The lipid denotes a glycerolipid, a glycerophospholipid, and a sphingolipid, which are described in the definition of LIPID MAPS (registered trademark). The lipid is at least one selected from a glycerolipid, a glycerophospholipid, or a sphingolipid. A glycerolipid, a glycerophospholipid, and a sphingolipid are preferable as the lipid.

Examples of the fatty acyl include fatty acids such as butyric acid, valeric acid, caproic acid, enanthic acid, caprylic acid, pelargonic acid, capric acid, lauric acid, myristic acid, pentadecylic acid, palmitic acid, palmitoleic acid, margaric acid, stearic acid, oleic acid, vaccenic acid, linoleic acid, linolenic acid, eleostearic acid, arachidic acid, mead acid, and arachidonic acid.

Examples of the glycerolipid include acylglycerol, glyceryl ether, and glyceroglycolipid. Among these, acylglycerol is preferable.

Examples of the glycerophospholipid include phosphatidylcholine and phosphatidylinositol.

Examples of the sphingolipid include sphingomyelin, a cerebroside, and a ganglioside.

The concentration of the sterol lipid contained in the culture medium A2 is 5.0 µg/mL or less, 4.5 µg/mL or less, 4.0 µg/mL or less, 3.5 µg/mL or less, or 3.0 µg/mL or less.

Examples of the sterol lipid include sterol, steroid hormones, and bile acid.

In a case where the culture medium A2 contains the fatty acyl, it is preferable that the culture medium A2 contain albumin since the fatty acyl is carried in a state of being bound to albumin.

Examples of the fatty acyl include fatty acids such as butyric acid, valeric acid, caproic acid, enanthic acid, caprylic acid, pelargonic acid, capric acid, lauric acid, myristic acid, pentadecylic acid, palmitic acid, palmitoleic acid, margaric acid, stearic acid, oleic acid, vaccenic acid, linoleic acid, linolenic acid, eleostearic acid, arachidic acid, mead acid, and arachidonic acid.

It is preferable that the culture medium A2 further contain a neurotrophic factor.

In a case where the culture medium A2 further contains a neurotrophic factor, the maturation of cells can be further promoted, and the survival rate of cells in the presence of the tau protein aggregate can be further improved.

Examples of the neurotrophic factor include a nerve growth factor (NGF), a brain-derived neurotrophic factor (BDNF), a glial cell line-derived neurotrophic factor (GDNF), neurotrophin-3 (NT-3), and neurotrophin-415 (NT-4/5). Among these, BDNF or GDNF is preferable, and a combination of these is more preferable.

The concentration of the neurotrophic factor in the culture medium can be set to be, for example, in a range of 1 ng/mL to 2000 µg/mL or in a range of 2 ng/mL to 1000 µg/mL. Further, in a case where two or more kinds of neurotrophic factors are used in combination, the total concentration of the neurotrophic factors is set to be in the above-described ranges and used.

The culture medium A2 can contain a carbon source such as an amino acid (500 ng/mL to 9000 µg/mL), an antioxidant (100 ng/mL to 200 µg/mL), a mineral (500 ng/mL to 9000 µg/mL), or glucose (300 ng/mL to 7500 µg/mL) and preferably a protein such as fatty acyl (100 ng/mL to 250 µg/mL), albumin, insulin, or transferrin (10 ng/mL to 100 µg/mL), an antibacterial agent (50 ng/mL to 350 µg/mL), and a reducing agent (100 ng/mL to 10 µg/mL) in addition to the above-described components.

The culture medium A2 is usually prepared by adding a lipid or a neurotrophic factor to a basal culture medium. The culture medium A2 can be prepared by further adding a culture medium supplement, an antibacterial agent, and the like.

The basal culture medium, the culture medium supplement, and the antibacterial agent described in the culture medium A1 can be used as the basal medium, the culture medium supplement, and the antibacterial agent. As a combination of the basal culture medium and the culture medium supplement, a combination of Neurobasal (registered trademark) culture medium (catalog number, "21103049", Thermo Fisher Scientific, Inc.) and "KnockOut Serum Replacement" (product name, manufactured by Thermo Fisher Scientific, Inc.) is preferable.

Suspension culture is preferable as the culture in step A2. Examples of the culture vessel used in the suspension culture include the cell non-adhesive culture vessel described in step A1.

The culture period in step A2 is usually 1 week or longer and preferably in a range of 3 weeks to 30 weeks.

The culture of step A1 is carried out in an environment where the temperature is usually in a range of 30°C to 50°C, preferably in a range of 32°C to 48°C, and more preferably in a range of 34°C to 46°C and the carbon dioxide content proportion is usually in a range of 1% by volume to 15% by volume, preferably in a range of 2% by volume to 14% by volume, and more preferably in a range of 3% by volume to 13% by volume. In addition, it is also possible to carry out culture in an atmosphere with a high oxygen concentration.

### <Step A1' and Step A2'>

The spheroid of human neural cell-like cells is cultured in the presence of tau-seeds in step A1' and cultured in a culture medium A2' containing a neurotrophic factor.

The culture conditions in step A I' are the same as those in step A1 and thus the description thereof will not be repeated.

Since the kind of the neurotrophic factor and the concentration of the neurotrophic factor in the culture medium in step A2', and the various culture conditions in step A2' are the same as those in step A2 described above, the detailed description thereof will not be repeated.

### <Human neural cell-like cell>

A human neural cell-like cell having a risk factor is preferable as the human neural cell-like cell. Examples of the risk factor include apolipoprotein gene ε4 genotype, mutations in MAPT, GRN, C9orf72, and the like, neprilysin knockdown, and internalization of hyperphosphorylated or ubiquitinated tau protein. Examples of the human neural cell-like cell having a risk factor include a specimen with neurofibrillary tangles (including a specimen from a sporadic patient and a familial patient), a culture product of a specimen with a genetic mutation, and a culture product (also referred to as "culture product a") obtained by maturing human neural cell-like cells having a mutation in MAPT genes. Among these, the culture product a is preferable as the human neural cell-like cell having a risk factor. Examples of a method for preparing the culture product a include a method including the following "step a". The human neural cell-like cell having a mutation in the MAPT gene obtained in step a can contain phosphorylated 3-repeat tau protein and phosphorylated 4-repeat tau protein at a ratio of about 1 to 1, and thus the human neural cell-like cell is a cell similar to a nerve cell in the brain of a patient with dementia.

Step a includes the following step 1.

Step 1 Is a step of carrying out suspension culture on human pluripotent stem cells (mutant human pluripotent stem cells, hereinafter, also referred to as "mhPSCs") having a genetic mutation in at least one or more base sequences in an exon selected from the group consisting of exon 9, exon 10, exon 11, exon 12, and exon 13 (hereinafter, also referred to as "exons 9 to 13") of the MAPT gene and at least one or more base sequences in intron 10.

A spheroid is formed by the suspension culture of step 1. Human neural cell-like cells having a mutation in the MAPT gene can be produced by inducing differentiation of the spheroid. Examples of a method for inducing differentiation of the spheroid include a method of culturing the spheroid in a culture medium 2 containing a Wnt signal transduction activator and an extracellular matrix (hereinafter, also referred to as "ECM") (step 2). Further, human neural cell-like cells having a mutation in the MAPT gene or a spheroid thereof can be obtained by culturing the culture product, which has been formed by the culture in step 2, in a culture medium 3 containing no ECM (step 3).

The mhPSCs are obtained by genetically modifying at least one or more base sequences (one or more bases) in exon 9 to exon 13 of the MAPT gene in the genome of the wild-type human pluripotent stem cells (hereinafter, also referred to as "hPSCs"), and at least one or more base sequences (one or more bases) in intron 10. Hereinafter, the genetically modified MAPT gene is also referred to as "the modified MAPT gene". The mhPSCs can be subjected to expansion culture.

Examples of the hPSCs include human embryonic stem cells (hESCs) and induced pluripotent stem cells (hiPSCs). Among these, hiPSCs are preferable.

The modified MAPT gene can be introduced into the genome of hPSCs by a genome editing technique using, for example, a transcription activating factor-like effector nuclease, a Zn finger nuclease, or CRISPR-Cas9. Among the above, CRISPR-Cas9 is preferable since a target gene can be inserted into a site of interest with high selectivity.

A CRISPR-Cas9 system is a system possessed by bacteria and archaebacteria, where the CRISPR-Cas9 system utilizes a locus that functions as one kind of acquired immunity against nucleic acids such as viral DNA, viral RNA, and plasmid DNA, which have invaded from the outside. In order to introduce the modified MAPT gene into a host DNA by the CRISPR-Cas9 system, a vector for cleaving an introduction site of the host DNA is prepared. For this purpose, an expression vector encoding a guide RNA that guides Cas9 to a site of interest and a Cas9 protein expression vector is required. In a case where these two kinds of expression vectors and a donor DNA containing the modified MAPT gene are introduced, the site of interest is cleaved, and then the modified MAPT gene is introduced by homologous recombination repair with the donor DNA. As the expression vector, it is possible to use one vector (one all-in-one vector) expressing both the guide RNA and the Cas9 protein instead of the two kinds of expression vectors.

It suffices that the sequence (the donor DNA) homologous to a site on the genome, the site being subjected to insertion, is operably linked upstream (the 5' side) or downstream (the 3' side) of the modified MAPT gene, and the length thereof is usually, 0.5 kb to 8 kb.

Examples of the method of introducing an expression vector or a donor DNA into hPSCs include a calcium phosphate method, an electroporation method, a lipofection method, an aggregation method, a microinjection method, a particle gun method, and a DEAE-dextran method.

Examples of the expression vectors include Escherichia coli-derived plasmids such as pBR322, pBR325, pUC12, pUCl3, and pUC57; Bacillus subtilis-derived plasmids such as pUB110, pTP5, and pC194; yeast-derived plasmids such as pSH19 and pSH15; bacteriophages such as λ phage; viruses such as adenovirus, adeno-associated virus, lentivirus, vaccinia virus, and baculovirus; and vectors obtained by modifying these.

The modified MAPT gene can be introduced together with a tag sequence, a promoter sequence, a transcription termination sequence, a drug selection marker gene, and a combination thereof. Examples of the tag sequence include a fluorescent tag, an affinity tag, a degron tag, a localization tag. In particular, in a case where the modified MAPT gene is allowed to have a drug selection marker, it is possible to efficiently select cells in which the modified MAPT gene has been introduced, by drug selection.

Examples of the drug selection marker gene include a neomycin resistance gene, a histidinol resistance gene, a puromycin resistance gene, a hygromycin resistance gene, a blasticidin resistance gene, an ampicillin resistance gene, and a chloramphenicol resistance gene. In a case where a drug selection marker gene is introduced into the tag sequence, the drug selection marker gene-introduced tag sequence can be removed after drug selection.

The MAPT gene (NCBI accession number: NG_007398.1) is present on human chromosome 17 long arm 17q21 and has 16 exons and 15 introns. The modified MAPT gene is a modified MAPT gene obtained by modifying at least one or more bases in exon 9 to exon 13 of the MAPT gene and at least one or more bases in intron 10.

A plurality of base sequence mutations of the MAPT gene are known to cause frontotemporal lobar degeneration associated with the accumulation of tau protein. Further, it is known that the mutation sites of base sequences of the MAPT gene are concentrated in the ranges of the base sequences in exon 9 to exon 13 and the base sequences in intron 10.

As the modified MAPT gene, it is possible to use a modified MAPT gene obtained by modifying at least one or more base sequences in exon 9 to exon 13 of the MAPT gene and at least one or more in intron 10. Among these, the number of base sequence mutations in exon 9 to exon 13 is preferably two or more and more preferably two. Further, the number of base sequence mutations in intron 10 is preferably one or more and more preferably one.

Examples of the base sequence mutation of the modified MAPT gene include a base sequence mutation in exon 9, such as a base sequence mutation encoding an amino acid mutation of K257V, K257T, 1260V, L266V, or G272V; a base sequence mutation in exon 10, such as a base sequence mutation encoding an amino acid mutation of N279K, ΔK280, L284L, N296N, N296H, ΔN296, P301L, P301S, P301T, G303V, S305N, or S305S; a base sequence mutation in exon 11, such as a base sequence mutation encoding an amino acid mutation of L315L, L315R, S320Y, S310F; a base sequence mutation in exon 12, such as a base sequence mutation encoding an amino acid mutation of V337M, E342V, G335V, G335S, Q336R, or K369I; and a base sequence mutation in exon 13, such as a base sequence mutation encoding an amino acid mutation of G389R or R406W. Further, examples of the base sequence mutation in intron 10 include E10 + 3, E10 + 11, E10 + 12, E10 + 13, E10 + 14, E10 + 16, and E10 + 19.

The base sequence mutation in exon 9 to exon 13 is preferably a base sequence mutation of exon 10, and the base sequence mutation in exon 10 is preferably N279K or P301S. The base sequence mutation in intron 10 is preferably E10 + 16. Human nerve cell-like cells in which misfolded tau proteins are accumulated can be prepared by using the modified MAPT gene on which the above-described modifications have been performed.

hiPSCs refers to cells in which pluripotency has been induced by reprogramming human somatic cells by a known method or the like.

Examples of the combination of reprogramming factors include a combination of Oct3/4, Sox2, Klf4, and Myc (c-Myc or L-Myc) and a combination of Oct3/4, Sox2, Klf4, Lin28, and L-Myc.

As the hiPSCs, hiPSCs established as cell lines can be used. The hiPSCs established as cell lines, such as a 201 B7 cell, a 201B7-Ff cell, a 253G1 cell, a 253G4 cell, a 1201C1 cell, a 1205D1 cell, a 1210B2 cell, and a 1231A3 cells, are available from Kyoto University or iPS Academia Japan, Inc. In addition, the hiPSCs established as cell lines, such as PCPhiPS771, are available from REPROCELL Inc. Further, the hiPSCs established as cell lines, such as XFiPS-F44-3F-2, are available from Institute of Health Carlos III.

The expansion culture is an operation of proliferating mhPSCs. In the expansion culture, it is preferable to carry out two-dimensional culture by adhesion culture. The adhesion culture is a culture carried out by allowing the adhesion between spheroids and the culture surface of a culture vessel. The expansion culture is usually carried out in an environment of 30°C to 50°C and a carbon dioxide content proportion of 1% by volume to 15% by volume.

As the culture vessel that is used in the adhesion culture, it is possible to use the same culture vessels as those described above, provided that the culture surface of the culture vessel is cell adhesive. Examples of the surface treatment for improving adhesiveness include coating of laminin such as laminin α5β1γ1, laminin α1β1γ1, laminin 511E8, entactin, collagen, gelatin, vitronectin, polylysine, or polyornithine, and positive charge treatment.

The culture medium that is used in the expansion culture (hereinafter, also referred to as an "expansion culture medium") is preferably a feeder-free culture medium. Examples of the feeder-free culture medium include known culture media such as an hES9 culture medium, an hES9a culture medium, and an hESF-FX culture medium, and commercially available product such as TeSR-E8 (product name, manufactured by STEMCELL Technologies) and StemFit (registered trademark).

In order to suppress cell death, the culture can be carried out in, as the expansion culture medium, a culture medium containing a ROCK inhibitor in the feeder-free culture medium before carrying out culture in the feeder-free culture medium. In a case where a culture medium obtained by adding a ROCK inhibitor to a feeder-free culture medium is used, the culture period may be at most 5 days, and then the culture is carried out in a feeder-free culture medium containing no ROCK inhibitor, usually for 1 day or more and preferably for 3 days or more.

Examples of the ROCK inhibitor include Y-27632 (CAS number: 129830-38-2), Fasudil/HA1077 (CAS number: 105628-07-7), H-1152 (CAS number: 871543-07-6), and Wf-536 (CAS number: 539857-64-2), as well as derivatives thereof.

The concentration of the ROCK inhibitor contained in the expansion culture medium is such that the concentration is usually 0.1 µM to 100 µM, the concentration is preferably 1 µM to 80 µM, and the concentration is more preferably 5 µM to 50 µM.

The spheroid of mhPSCs can be dispersed before expansion culture is performed. The dispersion refers to separating cells into a cell population of 100 or less, preferably a cell population of 50 or less, and more preferably a single cell by a dispersion treatment such as an enzyme treatment or a physical treatment. Examples of the dispersion treatment include a mechanical dispersion treatment, a cell dispersion liquid treatment, and a treatment of adding a cell protecting agent. These treatments can be combined. Among these, a cell dispersion liquid treatment is preferable.

Examples of the cell dispersion liquid that is used in the cell dispersion liquid treatment include a solution containing any of enzymes such as trypsin, collagenase, hyaluronidase, elastase, pronase, DNase, and papain; and a chelating agent such as ethylenediaminetetraacetic acid. Examples of the commercially available cell dispersion liquid include TrypLE Select and TrypLE Express manufactured by Thermo Fisher Scientific, Inc., and Nerve Cell Dispersion Liquid A (catalog number: SBMBX0801D-2A) manufactured by KAC Co., Ltd. Examples of the mechanical dispersion treatment include a pipetting treatment and a scraping operation with a scraper.

Before the dispersion treatment, a treatment with a cell protecting agent can be carried out to prevent cell death. Examples of the cell protecting agent include fibroblast growth factor (hereinafter, also referred to as "FGF"), heparin, a ROCK inhibitor, an insulin-like growth factor (hereinafter, also referred to as "1GF"), serum, and a serum substitute.

In step 1, the mhPSCs are subjected to suspension culture in the culture medium 1 to form a spheroid. Hereinafter, the spheroid formed by the suspension culture of step 1 is also referred to as a spheroid 1. Examples of the culture vessel used in step 1 include the same culture vessel as the cell non-adhesive culture vessel described in step A1.

It is preferable that the culture medium 1 contain a BMP signal transduction pathway inhibitor associated with the phosphorylation of Smad1/5/9 and a TGF-β signal transduction pathway inhibitor associated with the phosphorylation of Smad2/3. Further, the culture medium 1 can contain a Wnt signal transduction pathway inhibitor.

The culture medium 1 is usually prepared by adding a BMP signal transduction pathway inhibitor, a TGF-β signal transduction pathway inhibitor, a Wnt signal transduction pathway inhibitor, or a combination thereof to a basal culture medium. Examples of the basal culture medium include the same basal culture medium as described in the culture medium A1.

Examples of the BMP signal transduction pathway inhibitor include chordin, noggin, follistatin, and dorsomorphin (6-[4-(2-pyrimidine-1-yl-ethoxy)phenyl]-3-pyridine-4-yl-pyrazolo[1,5-a]pyrimidine), DMH1 (4-[6-(4-isopropoxyphenyl)pyrazolo[1,5-a]pyrimidine-3-yl]quinoline, 4-[6-[4-1-methylethoxy)phenyl]pyrazolo[1,5-a]pyrimidine-3-yl]-quinoline), and LDN193189 (4-(6-(4-(piperidine-1-yl)phenyl)pyrazolo[1,5-a]]pyrimidine-3-yl)quinoline. Among these, dorsomorphin or LDN193189 is preferable.

The concentration of the BMP signal transduction pathway inhibitor contained in the culture medium 1 is usually 0.5 µM to 10 µM, preferably 0.75 µM to 5 µM, and more preferably 1 µM to 3 µM.

The TGF-β signal transduction pathway inhibitor is a substance that inhibits a signal transduction pathway transduced by the phosphorylation of Smad2/3, and it is preferably a substance that selectively inhibits the kinase activity of the TGF-βI type receptor kinase (ALK5). Examples of the TGF-β signal transduction pathway inhibitor include A83-01 (CAS number: 909910-43-6), SB-431542 (CAS number: 301836-41-9), SB-505124 (CAS number: 694433-59-5), SB-525334 (CAS number: 356559-20-1), LY364947 (CAS number: 396129-53-6), SC-203294 (CAS number: 627536-09-08), SD-208 (CAS number: 627536-09-8), and SJN2511 (CAS number: 446859-33-2). Among these, A83-01 of SB-431542 is preferable.

The concentration of the TGF-β signal transduction pathway inhibitor contained in the culture medium 1 is usually 0.5 µM to 10 µM, preferably 0.75 µM to 5 µM, and more preferably 1 µM to 3 µM.

Examples of the Wnt signal transduction pathway inhibitor include IWR-1-endo(4-[(3aR,4S,7R,7aS)-1,3,3a,4,7,7a-hexahydro-1,3-dioxo-4,7-methano-2H-isoindol-2-yl]-N-8-quinolinyl-benzamide), IWP-2, XAV939, Dkk1, a Cerberus protein, a Wnt antibody, and a casein kinase inhibitor.

The concentration of the Wnt signal transduction pathway inhibitor contained in the culture medium 1 is usually in a range of 0.1 µM to 10 µM, preferably in a range of 0.5 µM to 5 µM, and more preferably in a range of 1 µM to 3 µM.

The culture medium 1 can further contain a culture medium supplement antibacterial agent and the like. The details of the culture medium supplement and the antibacterial agent are the same as the details of the culture medium supplement and the antibacterial agent described in the culture medium A1 above.

The culture period of step 1 is usually 1 day or more, preferably 3 to 14 days, and more preferably 4 to 12 days.

The culture of step 1 is carried out in an environment in which the temperature is usually 30°C to 50°C, preferably 32°C to 48°C, and more preferably 34°C to 46°C, and the carbon dioxide content proportion is usually 1% by volume to 15% by volume, preferably 2% by volume to 14% by volume, and more preferably 3% by volume to 13% by volume. In addition, it is also possible to carry out culture in an atmosphere with a high oxygen concentration.

Before carrying out the suspension culture in the culture medium 1, it is possible to disperse the spheroids of mhPSCs, which are formed by expansion culture. The treatment method for dispersion is the same as the treatment method for dispersion described in the expansion culture.

In step a, the human nerve cell-like cells having a mutation in the MAPT genes can be prepared by inducing differentiation of the culture product (spheroid 1) formed by the suspension culture in step 1. Examples of a method for inducing differentiation include a method including step 2 of culturing the spheroid 1 in the culture medium 2 containing a Wnt signal transduction activator and ECM.

Step 2 may be continuously carried out by replacing the culture medium 1 with the culture medium 2 without taking out the spheroid 1 from the culture medium 1 or can be carried out by taking out the spheroid 1 from the culture medium 1 and seeding it in the culture medium 2. The culture of step 2 is preferably a suspension culture.

The culture period of the culture of step 2 is usually 1 day or more, preferably 3 to 14 days, and more preferably 4 to 12 days.

The culture of step 2 is carried out in an environment in which the temperature is usually 30°C to 50°C, preferably 32°C to 48°C, and more preferably 34°C to 46°C, and the carbon dioxide content proportion is usually 1 % by volume to 15% by volume, preferably 2% by volume to 14% by volume, and more preferably 3% by volume to 13% by volume. In addition, it is also possible to carry out culture in an atmosphere with a high oxygen concentration.

The culture vessel that is used in the culture of step 2 is preferably a culture vessel of which the culture surface is a cell non-adhesive culture vessel, which is the same as that in step 1. The details of the cell non-adhesive culture vessel are the same as those in step 1.

The culture medium 2 is a culture medium containing ECM. Examples of the method of carrying out culture in a culture medium containing ECM include a method of embedding the spheroid 1 in ECM and carrying out culture thereof and a method of culturing the spheroid 1 in a culture medium mixed with ECM. Among these, a method of culturing the spheroid 1 in a culture medium mixed with ECM is preferable.

The culture medium mixed with ECM is prepared by carrying out mixing so that the volume of ECM is usually 1% by volume or more, preferably 5% by volume to 100% by volume, and more preferably 10% by volume to 90% with respect to the volume of components other than the ECM in the culture medium, and then gelating an ECM precursor. Examples of the method of mixing the ECM precursor and the components other than ECM include a method of carrying out pipetting on an ice bath. The culture medium mixed with ECM refers to a medium having the ECM that is not visually observed in the culture medium.

The culture medium 2 is usually prepared by adding ECM or a ECM precursor to a basal culture medium. Examples of the basal culture medium include the same basal culture medium as described in the culture medium A1.

Examples of the ECM include a component contained in a basal membrane and a glycoprotein present in an intercellular space. Examples of the component contained in the basement membrane include type IV collagen, laminin, heparan sulfate proteoglycan, and entactin. As the ECM, a commercially available product containing ECM can be used. Examples of the glycoprotein present in the intercellular space include collagen, laminin, entactin, fibronectin, and heparin sulfate. Examples of the commercially available product containing ECM include Matrigel (product name, manufactured by Corning Incorporated) and human type laminin (product name, manufactured by Sigma-Aldrich Co., LLC).

Matrigel contains basement membrane components derived from Englbreth Holm Swarm mouse sarcoma. The main components of the Matrigel are type IV collagen, laminin, heparan sulfate proteoglycan, and entactin; however, in addition to these, they include various growth factors such as epidermal growth factor (hereinafter, also referred to as "EGF"), nerve growth factor (hereinafter, also referred to as "NGF"), platelet-derived growth factor (hereinafter, also referred to as "PDGF"), and insulin-like growth factor 1 ((hereinafter, also referred to as "IGF-1"), as well as TGF-β. There is Matrigel that is low in grade in terms of the concentrations of these components, and in such Matrigel, the concentration of EGF is less than 0.5 ng/mL, the concentration of NGF is less than 0.2 ng/mL, the concentration of PDGF is less than 5 pg/mL, the concentration of IGF-1 is less than 5 ng/mL, and the concentration of TGF-β is less than 1.7 ng/mL. As the Matrigel, it is preferable to use Matrigel that is low in grade in terms of the content proportion of these components.

The culture medium 2 contains a Wnt signal transduction activator. Examples of the Wnt signal transduction activator include a GSK-3β inhibitor, a Wnt protein, and an LGR5 agonist. Among these, a GSK-3β inhibitor or a Wnt protein is preferable. The final concentration of the Wnt signal enhancing agent contained in the culture medium 2 is usually 0.1 µM to 10 µM, and preferably 0.2 µM to 5 µM.

Examples of the GSK-3P inhibitor include CHIR99021 (CAS number: 252917-06-9), kenpaullone (CAS number: 142273-20-9), and 6-bromoindirubin-3'-oxime (BIO, CAS number: 667463-62-9). Among these, CHIR99021 is preferable.

The Wnt protein is preferably a Wnt protein derived from mammals. Examples of the mammalian Wnt protein include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16. Among these, Wnt3a is preferable. The Wnt protein is more preferably a complex with afamin.

Examples of the LGR5 agonist include the R-spongin family. Examples of the R-spondin family include R-spondin-1, R-spondin-2, R-spondin-3, R-spondin-4, and the like, and among these, R-spondin-1 is preferable.

In a case where CHIR99021 is used as the Wnt signal transduction activator, the final concentration of CHIR99021 contained in the culture medium 2 is usually in a range of 0.1 µM to 30 µM and preferably in a range of 0.2 µM to 20 µM.

In a case where Wnt3a is used as the Wnt signal transduction activator, the final concentration of Wnt3a contained in the culture medium 2 is usually in a range of 0.1 ng/mL to 20 ng/mL and preferably in a range of 0.2 ng/mL to 10 ng/mL.

The culture medium 2 can contain a TGF-β signal transduction pathway inhibitor. Examples of the TGF-β signal transduction pathway inhibitor include the same ones as those in the culture medium 1 described above. Among these, SB-431542 or SC-203294, which can selectively inhibit the kinase activity of ALK5, is preferable. The final concentration of the TGF-β signal transduction pathway inhibitor contained in the culture medium 2 is usually 0.1 to 20 µM and preferably 0.1 µM to 10 µM.

The culture medium 2 can further contain a culture medium supplement, an antibacterial agent, and the like. Examples of the culture medium supplement and the antibacterial agent include the same as those described in the culture medium A1.

Step 3 is a step of culturing the culture product, which has been formed by the culture in step 2, in the culture medium 3 containing no ECM to obtain a spheroid of human neural cell-like cells having a mutation in the MAPT gene.

Step 3 may be carried out continuously by replacing the culture medium 2 with the culture medium 3 without taking out the culture preparation of step 2 from the culture medium 2 or can be carried out by taking out the culture preparation of step 2 from the culture medium 2 and seeding the culture preparation in the culture medium 3. The culture of step 3 is preferably suspension culture, and the suspension culture is preferably carried out while stirring the culture medium.

The culture period of step 3 is usually 1 day or more, preferably 2 days to 700 days, and more preferably 10 days to 365 days. The culture of step 3 is carried out in an environment in which the temperature is usually 30°C to 50°C, preferably 32°C to 48°C, and more preferably 34°C to 46°C, and the carbon dioxide content proportion is usually 1% by volume to 15% by volume, preferably 2% by volume to 14% by volume, and more preferably 3% by volume to 13% by volume. In addition, it is also possible to carry out culture in an atmosphere with a high oxygen concentration.

The culture vessel that is used in the culture of step 3 is preferably a cell non-adhesive culture vessel. The details of the cell non-adhesive culture vessel are the same as those in step 1 described above.

The culture medium 3 does not contain ECM. "Does not contain ECM" means that ECM is not intentionally added to the culture medium 3, and thus ECM mixed as an unavoidable impurity, which has a volume of ECM of 5% by volume or less with respect to the volume of the components in the culture medium, is allowed.

The culture medium 3 is usually prepared by adding a culture medium supplement, an antibacterial agent, and the like to a basal culture medium. Examples of the basal culture medium and the culture medium supplement include the same basal culture medium and the same culture medium supplement as described in the culture medium A1.

The culture product formed by the culture in step 3 is usually a spheroid of human neural cell-like cells having a mutation in the MAPT gene, but the spheroid can be formed again after dispersion of the spheroid. Examples of the dispersion treatment for the spheroid include a mechanical dispersion treatment, a cell dispersion liquid treatment, and a cell protecting agent addition treatment, and the details thereof are the same as the details of the dispersion of mhPSCs.

Examples of the method of forming the spheroid include a method of carrying out suspension culture in the culture medium 3 to form a spheroid. It is preferable that the culture vessel that is used in the suspension culture is a culture vessel of which the culture surface is cell non-adhesive, which is similar to that described above.

### <<Culture product>>

The culture product according to the present embodiment can be produced by the culture method described above.

The culture product according to the present embodiment is a human nerve cell-like cell in which misfolded tau proteins are accumulated. Since misfolding of the tau proteins is accelerated by phosphorylation or the like, the culture product according to the present embodiment contains preferably phosphorylated 3-repeat tau protein and more preferably phosphorylated 3-repeat tau protein and phosphorylated 4-repeat tau protein at a ratio of about 1 to 1 in the cells. The misfolded tau protein may be in a state of a fibril or an oligomer.

The culture product according to the present embodiment may be in the form of a spheroid containing human nerve cell-like cells in which misfolded tau proteins are accumulated in the cells. The spheroid can contain neural cell-like cells other than human nerve cell-like cells, such as glial cell-like cells. The above-described glial cell-like cells can contain a misfolded tau protein. Further, the spheroid may be a brain organoid that has been three-dimensionally self-organized.

An anti-MC 1 antibody specifically binds to a misfolded tau protein. Therefore, the presence of the misfolded tau protein can be detected by immunostaining with the anti-MC1 antibody or the like.

The tau protein in the human brain is expressed, by alternative splicing, as six kinds of isoforms consisting of 352 to 441 amino acids, having molecular weights different from each other.

Examples of the six kinds of isoforms include a 0N3R tau protein (352 amino acids, NCBI accession number: NP_058525.1, NM_016841.4, or the like), a 0N4R tau protein which has exons 2 and 3 (383 amino acids, NCBI accession number: NP_058518.1, NM_016834.4, or the like), a 1N3R tau protein having exon 2 (381 amino acids, NCBI accession number: NP_001190180.1, NM_001203251.1, or the like), a 1N4R tau protein having exon 2 (412 amino acids, NCBI accession number: NP_001116539.1, NM_001123067.3, or the like), a 2N3R tau protein having exons 2 and 3 (410 amino acids, NCBI accession numbers: NP_001190181.1, NM_001203252.1, or the like), and a 2N4R tau protein having exons 2 and 3 (441 amino acids, NCBI accession numbers: NP_005901.2, NM_005910.5, or the like).

The phosphorylated 4-repeat tau protein contained in the human nerve cell-like cells in the culture product according to the present embodiment preferably includes a phosphorylated 0N4R tau protein, and the phosphorylated 3-repeat tau protein preferably includes a phosphorylated 0N3R tau protein.

### <<Spheroid of human nerve cell-like cells>>

The spheroid of the human nerve cell-like cells according to the present embodiment contains human nerve cell-like cells containing MC1-positive tau protein in the cells. The spheroid of the human nerve cell-like cells according to the present embodiment can contain neural cell-like cells other than the human nerve cell-like cells, such as glial cell-like cells. The above-described glial cell-like cells can contain a misfolded tau protein. Further, the spheroid may be a brain organoid that has been three-dimensionally self-organized.

The content proportion of the number of MC1-positive human nerve cell-like cells in the total number of cells in the spheroid is usually in a range of 1% to 100% by cell number, more preferably in a range of 2% to 90% by cell number, and still more preferably in a range of 3% to 80% by cell number.

The spheroid of the human nerve cell-like cells according to the present embodiment can be prepared by the above-described culture method.

### <<Method for screening test substance>>

A method for screening a test substance according to the present embodiment includes bringing the culture product according to the present embodiment or the spheroid of human nerve cell-like cells according to the present embodiment into contact with the test substance and evaluating the influence of the human nerve cell-like cells (hereinafter, also referred to as "step X").

In step X, examples of the test substance include a natural compound library, a synthetic compound library, an existing drug library, a metabolite library, and a biological sample such as a brain disruption fluid, a cerebrospinal liquor, or a brain interstitial fluid. The existing drugs include, for example, AZD2858 and a methylthioninium chloride hydrate. In addition, a new drug can be used as the test substance.

In step X, the influence of the test substance on the culture product according to the present embodiment or the spheroid of the human nerve cell-like cells according to the present embodiment can be examined or evaluated by Western blotting, ELISA, immunostaining, or the like.

Further, a step of transplanting the culture product according to the present embodiment or the spheroid of human nerve cell-like cells according to the present embodiment into a brain of a mammal (hereinafter, also referred to as "step x") can be provided before step X. The drug efficacy of the test substance can be evaluated in an environment similar to that of a living body suffering from Alzheimer's disease by providing step x.

### [Examples]

Hereinafter, the present embodiment will be described more specifically based on Examples; however, the present embodiment is not intended to be limited to these Examples. All experiments were carried out based on the ethics research plan approved by Ethics Committee, Keio University School of Medicine.

### [Experimental Example 1]

### (Preparation of hiPSCs (mhiPSCs) into which modified MAPT gene had been introduced)

A guide RNA for targeting a target sequence of the MAPT gene was designed using a guide RNA design software "CRISPRdirect" for genome editing.

An oligonucleotide encoding the designed guide RNA was synthesized, and this oligonucleotide was inserted into the BbsI site of pSpCas9(BB)-2A-Puro (PX459) V2.0, which is an all-in-one vector for expressing both a Cas9 protein and a guide RNA.

A vector in which 1.5 kb on the 5' side was set as the 5' arm and 4 kb on the 3' side was set as the 3'arm in the vicinity of the target sequence of the MAPT gene and had been ligated to a puromycin-resistant cassette sandwiched by piggyBac inverted terminal repeats was used as a targeting vector. The targeting vector had a base mutation in exon 10, encoding amino acid mutations of N279K and P301S of the MAPT gene, and a mutation of E10 + 16 in intron 10.

Next, 10 µg of the pSpCas9(BB)-2A-Puro (PX459) V2.0 plasmid and 10 µg of the targeting vector were introduced into 1 million hiPSCs (PChiPS771 strain, Lot No.: A01QM28, manufactured by ReproCELL Inc.) by an electroporation method using a NEPA21 electroporator (Nepa Gene Co., Ltd.).

From the 2nd to the 12th day after the start of gene introduction, the selection was carried out with puromycin. Next, puromycin-resistant colonies were selected and subjected to genotyping by genomic DNA extraction and PCR.

Next, a negative selection was carried out in order to remove the puromycin resistant cassette, and colonies from which the puromycin resistant cassette was removed were selected. The genomic DNA was extracted from the obtained colonies with a commercially available kit (product name: "DNeasy Blood & Tissue Kit", QIAGEN N.V.), and the removal of the puromycin cassette was confirmed by the electrophoresis after PCR amplification. In addition, it was confirmed by the Sanger sequence analysis that the modified MAPT gene is included, and then mhiPSCs were obtained, using the extracted genomic DNA.

### [Experimental Example 2]

### (Expansion culture of mhiPSCs)

The mhiPSCs prepared in Experimental Example 1 were subjected to feeder-free culture. Specifically, the mhiPSCs were washed with phosphate buffered saline (PBS) and then dispersed into single cells using TrypLE Select (manufactured by Thermo Fisher Scientific, Inc.). Subsequently, the dispersed mhiPSCs were seeded in a plastic culture dish coated with a human recombinant laminin fragment (product name: "iMatrix-511", manufactured by Nippi. Inc.) containing only the active site of laminin-511 and subjected to feeder-free culture in a StemFit AK02N culture medium (manufactured by Ajinomoto Co., Inc.) in the presence of Y27632 (a ROCK inhibitor, 10 µM). In a case where a 60 mm dish (for cell culture, manufactured by IWAKI & CO., LTD.) was used as the plastic culture dish, the number of seeded cells of the hiPSCs dispersed into single cells was 3×10⁴ cells/dish.

One day after seeding the cells, the culture medium was exchanged with a StemFit AK02N culture medium containing no Y27632. Thereafter, the culture medium was exchanged with the StemFit AK02N culture medium containing no Y27632 once every 1 to 2 days. Thereafter, the cells were 80% confluent 6 days after seeding the cells.

### [Experimental Example 3]

### (Preparation of spheroid containing human neural cell-like cells having mutation in microtubule-associated protein tau gene)

An enlarged culture product of the mhiPSCs obtained in Experimental Example 2 was subjected to a cell dispersion liquid treatment using TrypLE Select (product name, manufactured by Thermo Fisher Scientific, Inc.) and further dispersed into single cells by a pipetting operation. The dispersed enlarged culture product of mhiPSCs was seeded in a 96-well culture plate (product name, "PrimeSurface 96V bottom plate", manufactured by Sumitomo Bakelite Co., Ltd.) in 100 µL/well of the culture medium 1 having the composition (excluding a BMP signal transduction pathway inhibitor) listed in Table 1 such that the cell density reached 1×10⁴ cells/well, and subjected to suspension culture in an incubator (37°C, 1 atm, CO₂ concentration: 5 v/v%) for 3 days, a BMP signal transduction pathway inhibitor listed in Table 1 was added thereto on the third day, and the culture product was further subjected to suspension culture for 4 days (7th day from the start of culturing mhiPSCs), thereby obtaining a spheroid of mhiPSCs.

Subsequently, 230 µL of the culture medium 1 was removed from the well. Subsequently, 150 µL/well of the culture medium 2 having the composition listed in Table 2 was added, and the spheroid was subjected to suspension culture while being stirred in an incubator (37°C, 1 atm, CO₂ concentration: 5 v/v%) (14th day from the start of culturing mhiPSCs) for 7 days.

Subsequently, the contents of the well were transferred to 50 mL Falcon (registered trademark) conical tube (manufactured by Corning Inc.) to which 10 mL of PBS was added. Subsequently, mixing with inversion was carried out 5 times, the culture medium 2 was removed by removing the supernatant, and the spheroids were collected. Thirty collected spheroids and the culture medium 3 (30 ml) having the composition listed in Table 3 were added to a single-use bioreactor (ABLE Corporation), and subjected to suspension culture while being stirred. The culture medium was exchanged every 4 days. The spheroids in the single-use bioreactor were collected on the 56th day after the start of the suspension culture (70th day after the start of culturing mhiPSCs).

**[Table 1]**

| Component | | Content |
|---|---|---|
| Basal culture medium | DMEM/F-12, HEPES (product name, manufactured by Thermo Fisher Scientific, Inc.) | - |
| Culture medium supplement | KnockOut^{™} Serum Replacement (product name, manufactured by Thermo Fisher Scientific, Inc.) | 25 v/v% with respect to basal culture medium |
| | MEM Non-Essential Amino Acids Solution (100X) (product name, manufactured by Thermo Fisher Scientific, Inc.) | Amount set such that dilution concentration reached 200 times that of basal culture medium |
| | Glutamax (product name, manufactured by Thermo Fisher Scientific, Inc.) | Amount set such that dilution concentration reached 100 times that of basal culture medium |
| | 2-Mercaptoethanol | Amount set such that dilution concentration reached 100 times that of basal culture medium |
| Antibacterial agent | Penicillin streptomycin mixed solution (product name, manufactured by NACALAI TESQUE, INC.) | Amount set such that dilution concentration reached 200 times that of basal culture medium |
| BMP signal transduction pathway inhibitor | LDN193189 | 200 nM |
| TGF-β signal transduction pathway inhibitor | SB-431542 | 10 µM |
| Wnt signal transduction pathway inhibitor | IWP-2 | 2 µM |

**[Table 2]**

| Component | | Content |
|---|---|---|
| Basal culture medium | DMEM/F-12, HEPES (product name, manufactured by Thermo Fisher Scientific, Inc.) | - |
| Culture medium supplement | MEM Non-Essential Amino Acids Solution (100X) (product name, manufactured by Thermo Fisher Scientific, Inc.) | Amount set such that dilution concentration reached 200 times that of basal culture medium |
| | Glutamax (product name, manufactured by Thermo Fisher Scientific, Inc.) | Amount set such that dilution concentration reached 100 times that of basal culture medium |
| Antibacterial agent | Penicillin streptomycin mixed solution (product name, manufactured by NACALAI TESQUE, INC.) | Amount set such that dilution concentration reached 200 times that of basal culture medium |
| Wnt signal activator | Wnt-3a, Human, Recombinant (product name, manufactured by R&D Systems) | 4 ng/mL |
| | CHIR99021 | 1 µM |
| TGF-β signal inhibitor | SB-431542 | 1 µM |
| Extracellular matrix | Matrigel | 30 v/v% |

**[Table 3]**

| Component | | Content |
|---|---|---|
| Basal culture medium | DMEM/F-12, HEPES (product name, manufactured by Thermo Fisher Scientific, Inc.) | - |
| Culture medium supplement | MEM Non-Essential Amino Acids Solution (100X) (product name, manufactured by Thermo Fisher Scientific, Inc.) | Amount set such that dilution concentration reached 200 times that of basal culture medium |
| | 2-Mercaptoethanol (product name, manufactured by Thermo Fisher Scientific, Inc.) | Amount set such that dilution concentration reached 1000 times that of basal culture medium |
| | Glutamax (product name, manufactured by Thermo Fisher Scientific, Inc.) | Amount set such that dilution concentration reached 100 times that of basal culture medium |
| | Chemically Defined Lipid Concentrate (product name, manufactured by Thermo Fisher Scientific, Inc.) | Amount set such that dilution concentration reached 100 times that of basal culture medium |
| | N2 Supplement (100X) (product name, manufactured by Thermo Fisher Scientific, Inc.) | Amount set such that dilution concentration reached 200 times that of basal culture medium |
| | B-27 (registered trademark) Serum Free Supplement (product name, manufactured by Thermo Fisher Scientific, Inc.) | Amount set such that dilution concentration reached 100 times that of basal culture medium |
| Antibacterial agent | Penicillin streptomycin mixed solution (product name, manufactured by NACALAI TESQUE, INC.) | Amount set such that dilution concentration reached 200 times that of basal culture medium |

### [Experimental Example 4]

### (Preparation of human nerve cell-like cells from wild-type hiPSCs)

Spheroids of human nerve cell-like cells were collected by performing the same operation as in Experimental Example 3 except that wild-type hiPSCs (PChiPS771 strain, manufactured by ReproCELL Inc.) were used in place of the enlarged culture product of mhiPSCs obtained in Experimental Example 2, in Experimental Example 3.

### [Experimental Example 5]

### (Preparation of human nerve cell-like cells in which misfolded tau proteins were accumulated by tau-seeding)

An increase in number of human nerve cell-like cells in which misfolded tau proteins were accumulated was evaluated using the spheroid obtained in Experimental Example 3 or Experimental Example 4 according to the experimental protocol listed in Table 4. "+" denotes that tau-seeds were present in the culture medium, and "-" denotes that tau-seeds were not present in the culture medium. Hereinafter, the operation procedure for "+" of "Mut" will be described.

**[Table 4]**

| | Spheroid obtained in Experimental Example 4 | | Spheroid obtained in Experimental Example 3 | |
|---|---|---|---|---|
| Tau-seed | - | + | - | + |
| Name of spheroid | WT | WT + seed | Mut | Mut + seed |

The spheroid obtained in Experimental Example 3 was subjected to a cell dispersion liquid treatment by using a dispersion liquid for nerve cells (product name, FUJIFILM Wako Pure Chemical Corporation) and further dispersed into single cells by performing a pipetting operation. The dispersion of the spheroid obtained in Experimental Example 3 was seeded in a 96-well culture plate (product name, "PrimeSurface 96V bottom plate", manufactured by Sumitomo Bakelite Co., Ltd.) in 100 µL/well of a culture medium 4 listed in Table 5 such that the cell density reached 1×10⁴ cells/well, and subjected to suspension culture in an incubator (37°C, 1 atm, CO₂ concentration: 5 v/v%) for 7 days, thereby forming a spheroid.

The culture medium 4 was removed, 100 µL/well of a culture medium 5 listed in Table 6 was added to the wells, 10 µL/well of Opti-MEM (manufactured by Thermo Fisher Scientific, Inc.) was added thereto, 0.2 µg/well of a fibril-type tau recombinant protein aggregate K18 type (trade code, "TAU02", manufactured by Cosmo Bio Co., Ltd.) and 0.2 µL/well of Lipofectamine 3000 (registered trademark, manufactured by Thermo Fisher Scientific, Inc.) were added thereto as tau-seeds, and suspension culture was carried out by setting the concentration of carbon dioxide in the vessel to 5% by volume at 37°C for 7 days. The fibril-type tau recombinant protein aggregate K18 type is an aggregate in which fragments of a human recombinant tau protein having a mutation of P301L in exon 10 of the human tau protein are aggregated.

Subsequently, after the culture for 7 days, the culture medium was exchanged with the fibril-type tau recombinant protein aggregate K18 type and a culture medium 6 containing no Lipofectamine 3000 and listed in Table 7, the suspension culture was continued for 70 days, and the spheroids were collected from the 96-well culture plate. The culture medium was exchanged every 7 days. The kind and the concentration of the lipid contained in the culture medium 6 are listed in Table 8.

**[Table 5]**

| Component | | Content |
|---|---|---|
| Basal culture medium | DMEM/F-12, HEPES (product name, manufactured by Thermo Fisher Scientific, Inc.) | - |
| Culture medium supplement | KnockOut^{™} Serum Replacement (product name, manufactured by Thermo Fisher Scientific, Inc.) | 25 v/v% with respect to basal culture medium |
| | MEM Non-Essential Amino Acids Solution (100X) (product name, manufactured by Thermo Fisher Scientific, Inc.) | Amount set such that dilution concentration reached 200 times that of basal culture medium |
| | Penicillin streptomycin mixed solution (product name, manufactured by NACALAI TESQUE, INC.) | Amount set such that dilution concentration reached 200 times that of basal culture medium |
| | Glutamax (product name, manufactured by Thermo Fisher Scientific, Inc.) | Amount set such that dilution concentration reached 100 times that of basal culture medium |
| | 2-Mercaptoethanol | Amount set such that dilution concentration reached 100 times that of basal culture medium |

**[Table 6]**

| Component | | Content |
|---|---|---|
| Basal culture medium | DMEM/F-12, HEPES (product name, manufactured by Thermo Fisher Scientific, Inc.) | - |
| Culture medium supplement | MEM Non-Essential Amino Acids Solution (100X) (product name, manufactured by Thermo Fisher Scientific, Inc.) | Amount set such that dilution concentration reached 200 times that of basal culture medium |
| | 2-Mercaptoethanol (product name, manufactured by Thermo Fisher Scientific, Inc.) | Amount set such that dilution concentration reached 1000 times that of basal culture medium |
| | Glutamax (product name, manufactured by Thermo Fisher Scientific, Inc.) | Amount set such that dilution concentration reached 100 times that of basal culture medium |
| | Chemically Defined Lipid Concentrate (product name, manufactured by Thermo Fisher Scientific, Inc.) | Amount set such that dilution concentration reached 100 times that of basal culture medium |
| | N2 Supplement (100X) (product name, manufactured by Thermo Fisher Scientific, Inc.) | Amount set such that dilution concentration reached 200 times that of basal culture medium |
| | B-27 (registered trademark) Serum Free Supplement (product name, manufactured by Thermo Fisher Scientific, Inc.) | Amount set such that dilution concentration reached 100 times that of basal culture medium |
| Antibacterial agent | Penicillin streptomycin mixed solution (product name, manufactured by Thermo Fisher Scientific, Inc.) | Amount set such that dilution concentration reached 200 times that of basal culture medium |

**[Table 7]**

| Component | | Content |
|---|---|---|
| Basal culture medium | Neurobasal^{™} Medium (product name, manufactured by Thermo Fisher Scientific, Inc.) | - |
| Culture medium supplement | KnockOut^{™} Serum Replacement (product name, manufactured by Thermo Fisher Scientific, Inc.) | 25 v/v% with respect to basal culture medium |
| | 2-Mercaptoethanol (product name, manufactured by Thermo Fisher Scientific, Inc.) | Amount set such that dilution concentration reached 1000 times that of basal culture medium |
| | Glutamax (product name, manufactured by Thermo Fisher Scientific, Inc.) | Amount set such that dilution concentration reached 100 times that of basal culture medium |
| | B-27 (registered trademark) Serum Free Supplement (product name, manufactured by Thermo Fisher Scientific, Inc.) | Amount set such that dilution concentration reached 50 times that of basal culture medium |
| Antibacterial agent | Penicillin streptomycin mixed solution (product name, manufactured by Thermo Fisher Scientific, Inc.) | Amount set such that dilution concentration reached 200 times that of basal culture medium |
| Neurotrophic factor | BDNF (product name, manufactured by PeproTech) | 100 µg/mL |
| | GDNF (product name, manufactured by PeproTech) | 100 µg/mL |

**[Table 8]**

| Kind and concentration of lipid | Culture medium 5 | Culture medium 6 |
|---|---|---|
| | Concentration (µg/mL) | Concentration (µg/mL) |
| Lysophosphatidylcholine | 0 | 18.1 |
| Triacylglycerol | 0 | 15.7 |
| Phosphatidylcholine | 0 | 8.5 |
| Phosphatidic acid | 0 | 3.3 |
| Sphingomyelin | 0 | 1.3 |
| Free fatty acid | 4.72 | 59.7 |
| Cholesterol | 2.2 | 1.4 |

### [Experimental Example 6]

### (Evaluation of isoform of tau protein)

Isoforms of the tau proteins contained in the human nerve cell-like cells of the spheroid collected in Experimental Example 5 were observed by the Western blotting method. An N-PER buffer (product name: "N-PER Neuronal Protein Extraction Reagent", manufactured by Thermo Fisher Scientific, Inc.) obtained by adding a phosphatase inhibitor to the spheroid was added, and ultrasonic disruption was performed on ice. Subsequently, the obtained cell disruption solution was allowed to stand on ice for 60 minutes. Subsequently, the cell disruption solution was centrifuged at 4°C and 15,000 × g, whereby the supernatant was collected. Subsequently, the protein concentration in the supernatant was measured using the bicinchoninic acid assay kit. Next, λ phosphatase was added to the supernatant, followed by a reaction at 30°C for 1.5 hours. Subsequently, the supernatant was applied to the wells, SDS-polyacrylamide gel electrophoresis (PAGE) was carried out, and the proteins were transferred to a polyvinylidene di-fluoride membrane (hereinafter, "PVDF membrane").

The PVDF membrane was incubated in a total protein staining solution (product name: "Revert 700 Total Protein Stain", manufactured by LI-COR, Inc.) at room temperature for 5 minutes, and washed twice with a washing solution (product name, "revert 700 Wash Solution", LI-COR, Inc.) at room temperature for 30 seconds, and total proteins were imaged using a fluorescence detection device (product name: "Odyssey Fc", manufactured by LI-COR, Inc.). After the imaging, the membrane was incubated in a destaining solution (product name, "Revert Destaining Solution", manufactured by LI-COR, Inc.) at room temperature for 10 minutes so that the membrane was destained, and rinsed with MQ. Thereafter, the membrane was immersed in a PVDF Blocking reagent (product name, "Intercept (TBS) Blocking Buffer", manufactured by LI-COR, Inc.), blocked at room temperature for 1 hour, and allowed to react with a reaction solution containing a mouse anti-tau antibody (tau5) and a rabbit anti-4-repeat tau antibody diluted to 1000 times and 2000 times, respectively, with a Blocking Buffer (product name, "Intercept (TBS) Blocking Buffer", manufactured by LI-COR, Inc.) containing 0.1% Tween-20 at 4°C overnight. Next, the membrane was washed with a TBST buffer four times and allowed to react, at room temperature for 1 hour, with a reaction solution containing a goat anti-mouse IgG antibody fluorescently labeled at a wavelength of 680 nm and a goat anti-rabbit IgG antibody fluorescently labeled at a wavelength of 800 nm, which had been diluted to 15000 times with a blocking buffer (product name, "Intercept (TBS) blocking buffer", manufactured by LI-COR, Inc.) to which Tween-20 (final concentration of 0.1%) and SDS (final concentration of 0.1%) had been added.

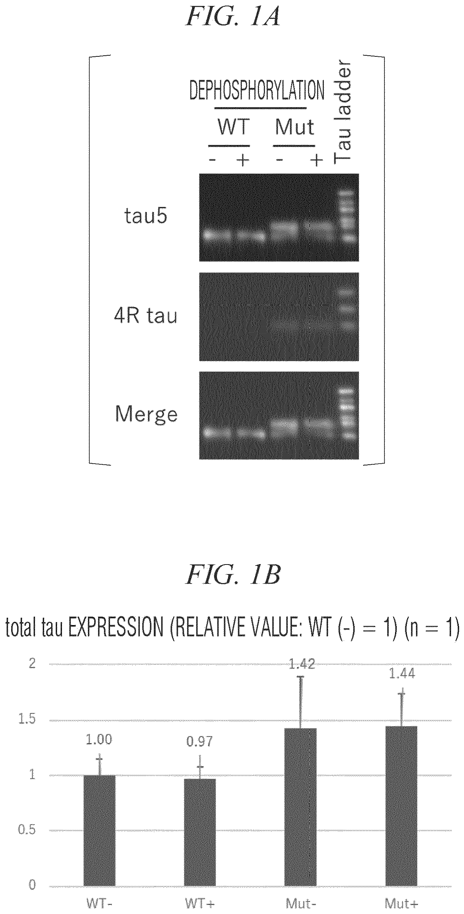

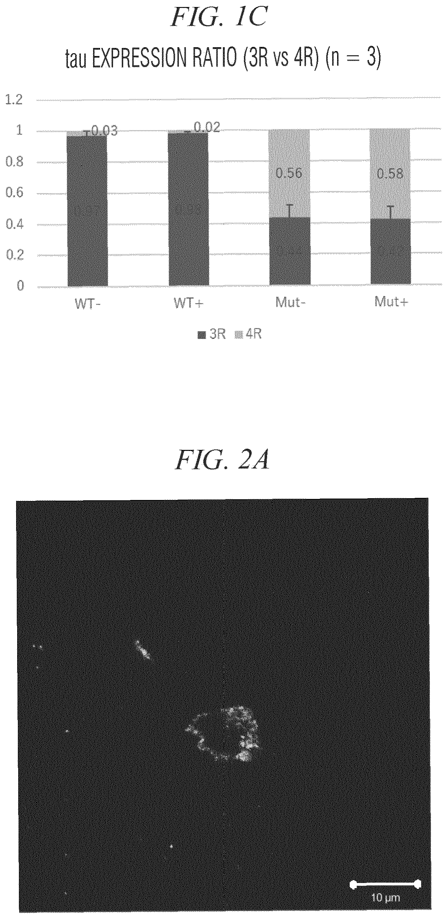

Subsequently, the membrane was washed with a TBST buffer four times and washed with a TBS buffer once. Thereafter, the bands of the isoforms of the tau proteins were detected using a fluorescence detection device (product name: "Odyssey Fc", manufactured by LI-COR, Inc.). FIG. 1A is photographs showing the results of Western blotting, and FIG. 1B and FIG. 1C are signal quantification graphs of Western blotting. The total protein signal was used for normalization.

The signals were quantified and normalized with the total protein signal. The band of 4R tau was confirmed only with Mut. The graphs in FIGS. 1B and FIG. 1C show the average and the standard deviation of n = 3. In FIGS. 1A to FIG. 1C, "Mut" denotes the result of the nerve cell-like cells of the spheroid obtained in Experimental Example 3, and "WT" denotes the result of the nerve cell-like cells of the spheroid obtained in Experimental Example 4.

The vertical axis of the Total tau expression graph represents a relative value in a case where the value of WT (-) is set to 1. Tau ladder is a recombinant tau containing six tau isoforms, where 0N3R, 0N4R, 1N3R, 1N4R, 2N3R, and 2N4R are shown from the bottom.

### [Experimental Example 7]

### (Immunostaining with MC1 antibody and RTM38 antibody)

The misfolded 3-repeat tau protein and the misfolded 4-repeat tau protein contained in the nerve cell-like cells of the spheroids was evaluated by fluorescent immunostaining of the sections of the spheroids (Mut + seed) obtained in Experimental Example 5 using an MC1 antibody. In addition, the presence or absence of tau proteins was evaluated using an RTM38 antibody. In addition, the nuclei were also stained with 4',6-diamidino-2-phenylindole (DAPI).

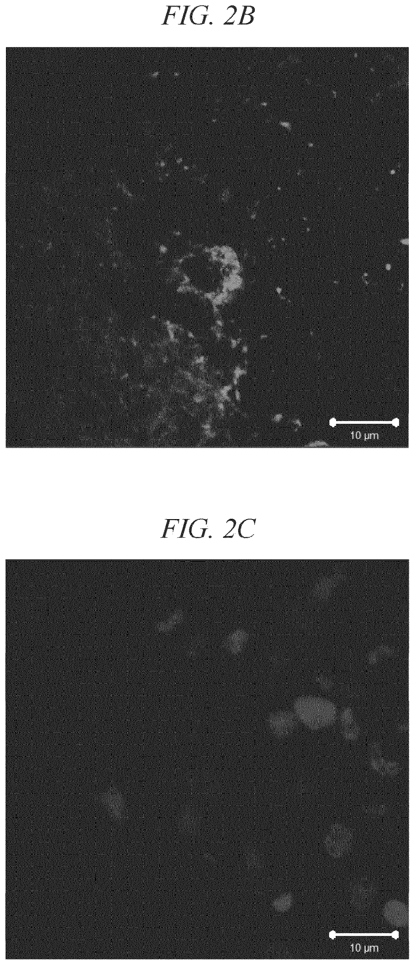

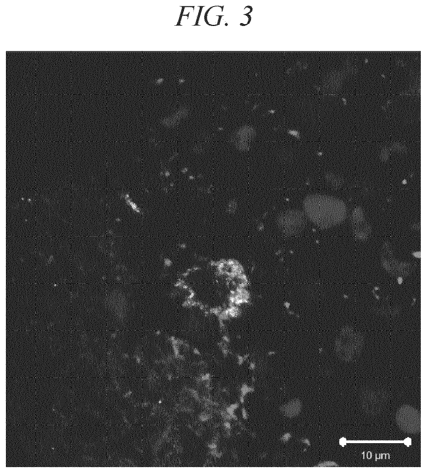

FIG. 2A shows a fluorescent immunostaining image using an MC1 antibody, FIG. 2B shows a fluorescent immunostaining image using an RTM38 antibody, and FIG. 2C shows a fluorescent immunostaining image using DAPl. Further, FIG. 3 shows fluorescent immunostaining images obtained by merging FIGS. 2A to 2C.

Since a signal derived from an MC1 antibody (antibody that recognizes the tau protein causing structural abnormality) and a signal derived from an RTM38 antibody (antibody that recognizes the C-terminal of the tau protein as an epitope and recognizes the misfolded tau protein without recognizing tau-seeds in the present culture system) overlapped with each other, it was found that the misfolded tau protein caused structural abnormality.

### [Experimental Example 8]

### (Immunostaining of sections of spheroids using TOMA antibody and ubi antibody)

The presence of tau oligomers contained in the nerve cell-like cells of the spheroids was confirmed by the fluorescent signals of the TOMA antibody in the sections of the spheroids (Mut and Mut + seed) obtained in Experimental Example 5. Further, the presence or absence of ubiquitination was evaluated using a ubi antibody.

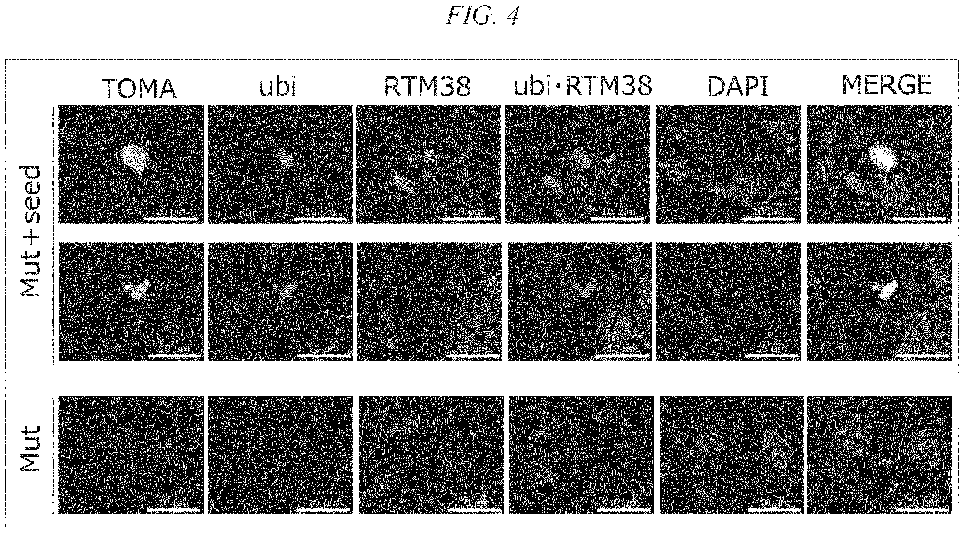

In FIG. 4, "TOMA" denotes a fluorescent immunostaining image using a TOMA antibody, "ubi" denotes a fluorescent immunostaining image using a ubi antibody, "RTM38" represents a fluorescent immunostaining image using an RTM38 antibody, "ubi·RTM38" denotes a fluorescent immunostaining image obtained by merging "ubi" and "RTM38", "DAPI" denotes a fluorescent immunostaining image using DAPI, and "MERGE" denotes a fluorescent immunostaining image obtained by merging "TOMA", "ubi", "RTM38", and "DAPI".

Since the fluorescence signal derived from a TOMA antibody (antibody that recognizes the tau oligomer and also recognizes the tau-seeds) and the signal derived from the ubiquitin antibody overlapped with each other, the tau-seeds were taken into the cells and underwent ubiquitination. Further, the signals overlapped with the signal derived from an RTM38 antibody in some cases, which suggests that the endogenous tau protein forms an oligomer and undergoes ubiquitination.

### [Experimental Example 9]

### (Immunostaining of sections of spheroids using T22 antibody)

The state of the MC1 antibody-positive tau contained in the nerve cell-like cells of the spheroids was evaluated by fluorescent immunostaining of sections of the spheroids (Mut and Mut + seed) obtained in Experimental Example 7, using a T22 antibody.

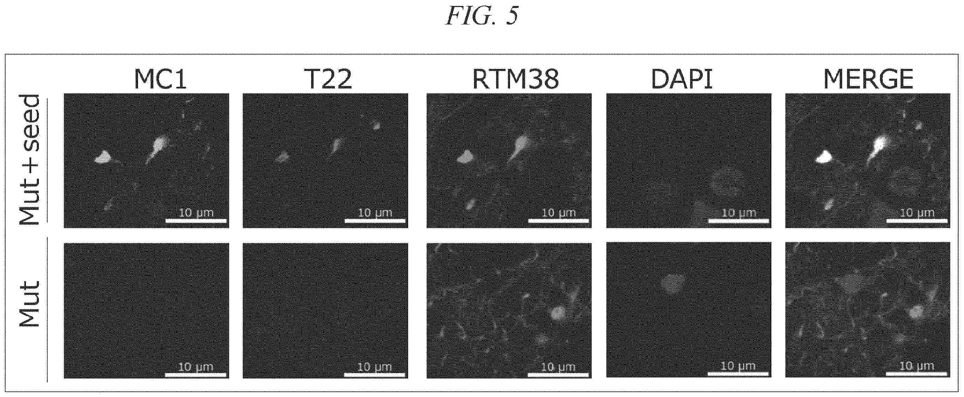

In FIG. 5, "MC1" denotes a fluorescent immunostaining image using an MC1 antibody, "T22" denotes a fluorescent immunostaining image using a T22 antibody, "RTM38" denotes a fluorescent immunostaining image using an RTM38 antibody, "DAPI" denotes a fluorescent immunostaining image using DAPI, and "MERGE" denotes a fluorescent immunostaining image obtained by merging "MCl ", "T22", "RTM38", and "DAPI".

Since the signal of the MCI-positive tau protein and the fluorescence signal derived from a T22 antibody (antibody that recognizes a tau oligomer and also recognizes tau-seeds) overlapped with each other, the structurally abnormal tau proteins formed oligomers.

### [Experimental Example 10]

### (Influence of culture medium composition in preparation of human nerve cell-like cells in which the misfolding of tau proteins were triggered by the exposure to tau-seeds)

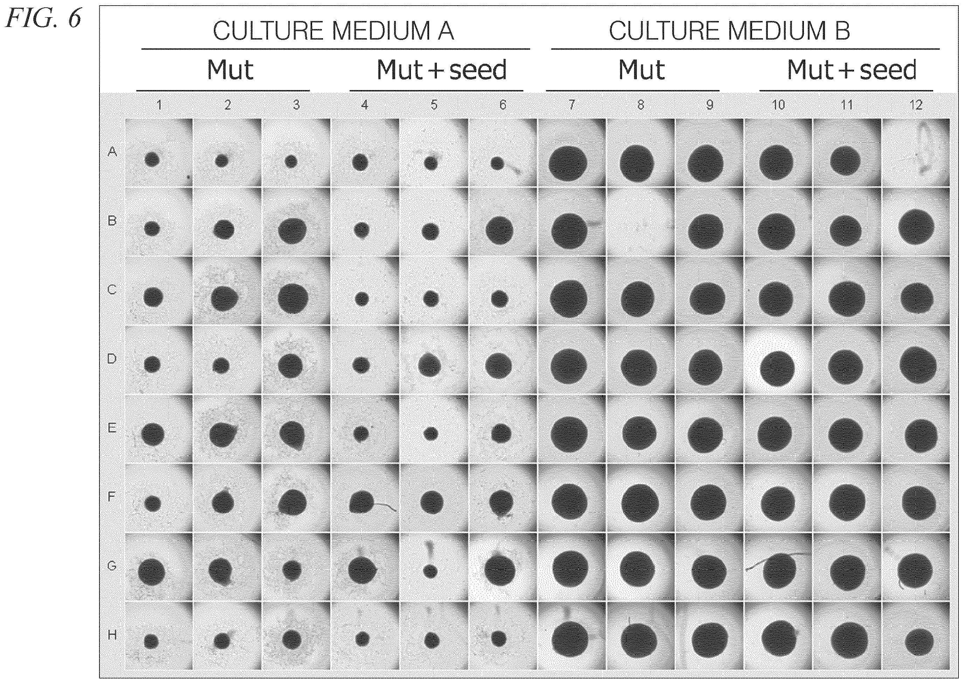

Human nerve cell-like cells in which misfolded tau proteins were accumulated, which was caused by tau-seeding, were prepared by the same operation as in Experimental Example 5 except that the culture medium 5 was used in place of the culture medium 6 in Experimental Example 5. FIG. 6 is an optical microphotograph showing the state of spheroids in a 96-well culture plate after the addition of tau-seeds and after being subjected to suspension culture for 8 weeks (3 × 8 = 24 images). The kinds and the concentrations of the lipids contained in the culture medium 5 are listed in Table 8.

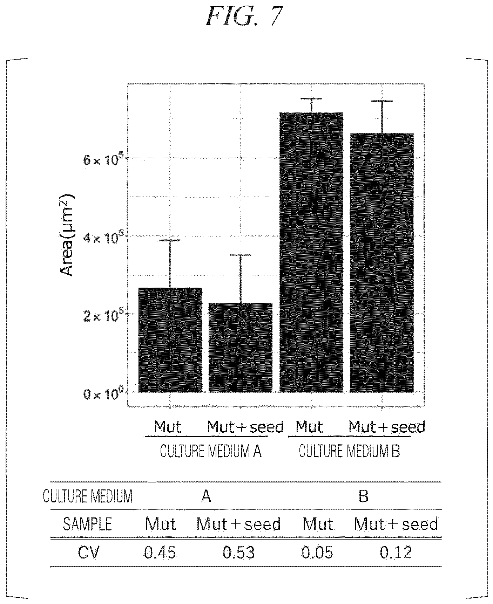

In FIG. 6, the culture medium A shows the results obtained by using the culture medium 5, and the culture medium B shows the results obtained by using the culture medium 6. Further, FIG. 7 is a graph showing an average value of the sizes of 24 spheroids and the coefficient of variation (CV) of the sizes of 24 spheroids.

It is considered that the culture medium containing lipids and a neurotrophic factor promotes the maturation of immature nerve cell-like cells and increases the number of cells thereof, which leads to the stabilization of culture.

### [Experimental Example 11]

### (Observation of tau fibers using immunoelectron microscope)

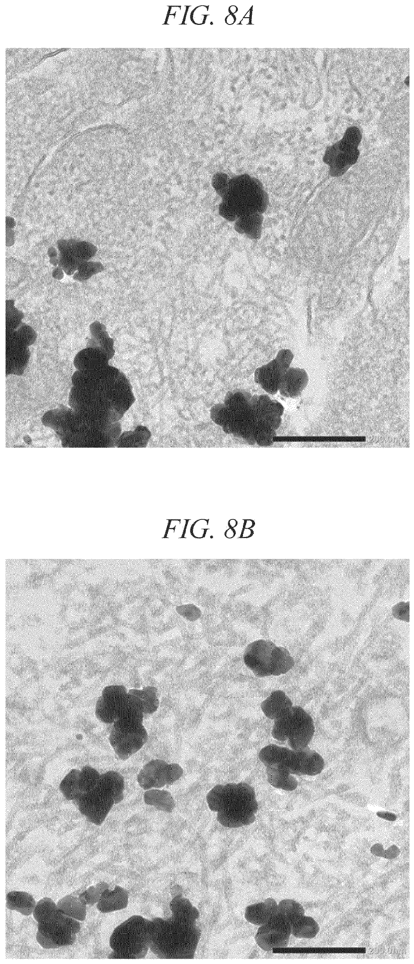

The spheroids (Mut + seed) collected in Experimental Example 5 and a brain section (obtained from BioChain) of an AD patient were observed with an electron microscope by preparing a sample for immunoelectron microscopy in conformity with the method described in the document (Front Neural Circuits. 2019, May 8; 13: 29). The photographs of immunoelectron microscopy are shown in FIG. 8A (Mut + seed) and FIG. 8B (brain section of the AD patient).

It was confirmed that in the spheroid obtained by the culture method according to the present embodiment, tau fibers formed by the aggregation of tau were present in the vicinity of the nerve cells, similar to a case of the brain section of the AD patient.

### [Experimental Example 12]

### (Evaluation of concentration of lipid contained in culture medium)

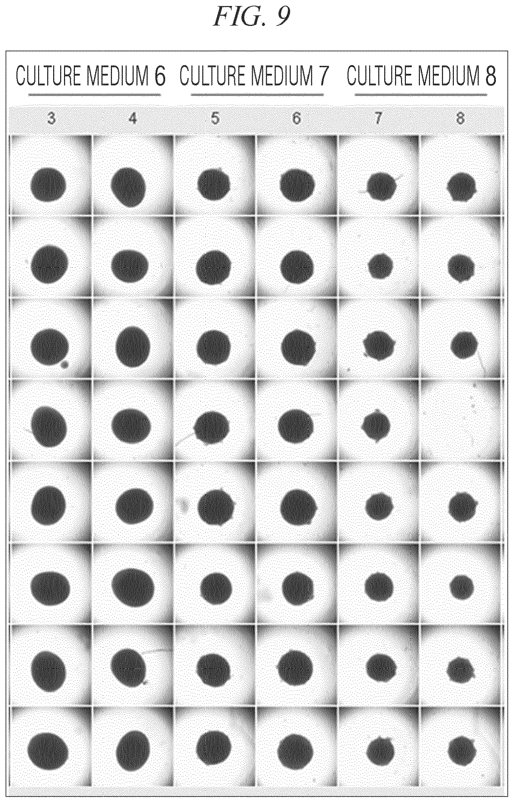

The culture was performed and the spheroids were collected by performing the same operation as in Experimental Example 5 except that the spheroid obtained in Experimental Example 3, a fibril-type tau recombinant protein aggregate K18 type (trade code, "TAU02", manufactured by Cosmo Bio Co., Ltd.), and Lipofectamine 3000 (registered trademark, manufactured by Thermo Fisher Scientific, Inc.) were used, the addition amounts of Neurobasal^{™} Medium (product name, manufactured by Thermo Fisher Scientific, Inc.) and KnockOut^{™} Serum Replacement (product name, manufactured by Thermo Fisher Scientific, Inc.) in the culture medium 6 were changed in place of the culture medium 6 listed in Table 7, and a culture medium 7 and a culture medium 8 of the kinds listed in Table 9 and containing lipids at the concentration listed in Table 9 were used. The culture results are listed in FIG. 9.

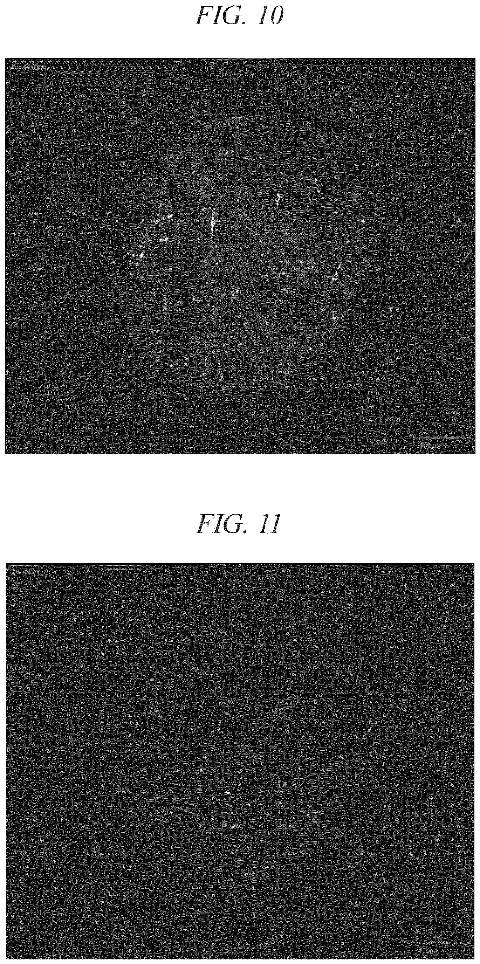

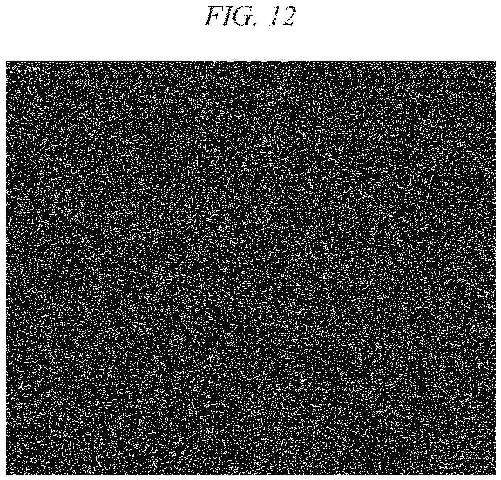

The spheroids were immunostained by performing the same operations as in Experimental Examples 7 to 9. The results are shown in FIGS. 10 to 12.

**[Table 9]**

| Kind and concentration of lipid | Culture medium 7 | Culture medium 8 |
|---|---|---|
| | Concentration (µg/mL) | Concentration (µg/mL) |
| Lysophosphatidylcholine | 36.2 | 54.3 |
| Triacylglycerol | 31.4 | 47.1 |
| Phosphatidylcholine | 17.0 | 25.5 |
| Phosphatidic acid | 6.6 | 9.9 |
| Sphingomyelin | 2.6 | 3.9 |
| Free fatty acid | 119.4 | 179.1 |
| Cholesterol | 2.8 | 4.2 |

### [Industrial Applicability]

According to the culture method of the present embodiment, it is possible to provide a culture method that enables efficient preparation of human nerve cell-like cells in which misfolded tau proteins are accumulated due to tau protein aggregates.

## Claims

1. A culture method comprising:
culturing a spheroid of a human neural cell-like cell in the presence of a tau protein aggregate and culturing a culture product in a culture medium containing 5 µg/mL or greater of a lipid,
wherein the lipid is one or more selected from the group consisting of a glycerolipid, a glycerophospholipid, and a sphingolipid.

2. The culture method according to Claim 1,
wherein the lipid is formed of a glycerolipid, a glycerophospholipid, and a sphingolipid.

3. The culture method according to Claim 1,
wherein a concentration of a sterol lipid contained in the culture medium is 5 µg/mL or less.

4. The culture method according to Claim 1,
wherein the lipid is one or more selected from the group consisting of triacylglycerol, lysophosphatidylcholine, phosphatidylcholine, phosphatidic acid, and sphingomyelin.

5. The culture method according to Claim 1,
wherein a concentration of the lipid contained in the culture medium is 5 µg/mL or greater and 150 µg/mL or less.

6. The culture method according to Claim 1,
wherein the culture medium further contains a neurotrophic factor.

7. A culture method comprising:
culturing a spheroid of a human neural cell-like cell in the presence of a tau protein aggregate and culturing a culture product in a culture medium containing a neurotrophic factor.

8. The culture method according to Claim 6 or 7,
wherein the neurotrophic factor is a brain-derived neurotrophic factor or a glial cell line-derived neurotrophic factor.

9. The culture method according to Claim 1 or 7,
wherein the human neural cell-like cell has a mutation in a microtubule-associated protein tau gene.

10. The culture method according to Claim 9,
wherein in the mutation, a mutation of a base sequence in an exon is a mutation of a base sequence that encodes one or more amino acid mutations selected from the group consisting of N279K, V337M, P301L, P301S, and R406W, and
a mutation of a base sequence in intron 10 is a mutation of one or more base sequences selected from the group consisting of E10+14 and E10+16.

11. The culture method according to Claim 1 or 7,
wherein the tau protein aggregate is an aggregate of a recombinant tau protein.

12. The culture method according to Claim 11,
wherein the recombinant tau protein is expressed from a microtubule-associated protein tau gene having a mutation of a base sequence of exon 10.

13. The culture method according to Claim 1 or 7,
wherein the human neural cell-like cell is a culture product.

14. A culture product cultured by the culture method according to Claim 1.

15. A culture product cultured by the culture method according to Claim 7.

16. A spheroid containing a human neural cell-like cell, comprising:
a MC1-positive tau protein in a cell.

17. A method for screening a test substance, comprising:
bringing the culture product according to Claim 14 of 15 of the spheroid containing a human neural cell-like cell according to Claim 16 into contact with the test substance and evaluating an influence of the human neural cell-like cell.
